# EUROPEAN PATENT SPECIFICATION

(11) **EP 2 857 491 B1**
(45) Date of publication and mention of the grant of the patent: **08.06.2016**
(21) Application number: 14187618.5
(22) Date of filing: 03.10.2014
(51) Int. Cl.: C11D 11/00, D06F 39/02, A47L 15/44, C11D 3/39

(54) **DEVICE FOR ON-SITE PREPARATION AND APPLICATION OF A BLEACH AND/OR DISINFECTANT, CORRESPONDING METHOD AND USE OF A FLOWMETER**
VORRICHTUNG ZUR VOR-ORT-HERSTELLUNG UND -VERWENDUNG EINES BLEICH- UND/ODER DESINFEKTIONSMITTELS, ENTSPRECHENDES VERFAHREN UND VERWENDUNG EINES DURCHFLUSSMESSERS
DISPOSITIF DE PRÉPARATION SUR SITE ET L'APPLICATION D'UN AGENT DE BLANCHIMENT ET/OU UN DÉSINFECTANT, MÉTHODE CORRESPONDANTE ET UTILISATION D'UN DÉBITMÈTRE

(30) Priority: 04.10.2013 BE 201300666
(43) Date of publication of application: 08.04.2015
(73) Proprietor: Christeyns N.V., 9000 Gent (BE)
(72) Inventor: Bostoen, Alain, Joseph, André, 9000 Gent (BE); Viry, Cécile Monique Paule, 8520 Kuurne (BE); De Meulemeester, Francky Julo, 8790 Waregem (BE)
(74) Representative: Patentwerk B.V.

(56) References cited:
- AT-U1- 6 023
- US-A1- 2002 061 263
- US-A1- 2007 231 220
- US-A1- 2012 172 437
- US-A1- 2013 098 450

## Description

The invention relates to on-site preparation and application of a bleach and/or disinfectant. The invention relates particularly to a device for on-site preparation and application of a bleach and/or disinfectant. The invention further relates to a method for on-site preparation and application of a bleach and/or disinfectant, in particular with the device according to the present invention, a system comprising a device according to the present invention and a treatment device for objects, in particular textile or food product, connected to the device, and the use of a flowmeter, particularly in a device according to the present invention.

Bleaches and disinfectants, in particular bleaches and disinfectants on the basis of peroxy acid (peracid), are used in diverse fields including the dairy, food and beverage processing industries as well as commercial laundries (including household laundry). Peracid-based sanitizing agents are also used in the treatment of cooling water, process water and domestic wastewater.

Peracid-based disinfectants and/or cleaning agents are in practice produced in bulk and generally supplied and stored in ventilated tanks until the peracid is used. Peracid is generally supplied as solution with a relatively low concentration of peracid varying between 5% and 35%. Determined solutions comprise a concentration of peracid which varies about round 1% or less. Such a solution of peracid, such as the much used peracetic acid (PAA), is generally obtained from an equilibrium reaction by mixing hydrogen peroxide, acetic acid, water and optionally an acid catalyst in water. Such solutions have the drawback that the equilibrium of such a reaction is relatively unfavourable, this meaning in practice that the peracid-based sanitizing agents are supplied including an excess of hydrogen peroxide and acetic acid in water.

Alternative methods of supplying peracid-based sanitizing agents and/or cleaning agents are also available. Conventional non-equilibrium solutions of peracid, such as PAA, are thus produced commercially in bulk by distilling acid equilibrium solutions of PAA and storing the obtained non-equilibrium distillate at a temperature of around freezing point in order to limit decomposition of PAA to a minimum during storage. However, this method of generating non-equilibrium solutions for PAA is not practical for smaller users due to the accuracy required during the preparation process, the use of concentrated hazardous substances and the explosion hazard occurring during the distillation of concentrated peroxides, such factors considerably increasing the cost price of such a distillation procedure.

A further alternative method is the preparation of peracid, such as PAA, by means of an irreversible reaction (non-equilibrium) wherein an acyl donor, such as triacetin in the case of the production of PAA, is used in combination with a peroxide, for instance hydrogen peroxide, and a base, for instance sodium hydroxide or potassium hydroxide.

A significant drawback of the above stated methods of preparing peracid such as PAA is that the peracid concentration decreases over time as a result of decomposition to acetic acid. In addition, the cleaning and sanitizing of installations used in for instance the food industry and the treatment of objects such as dairy, food and beverage products and textiles is a non-continuous process wherein a bulk-produced peracid solution will only be partially used. The application of bleaches and/or disinfectants for treating objects, where peracid-based sanitizing agents are produced, supplied and stored in bulk until the moment that the peracid is used, is therefore exceptionally inefficient.

The present invention therefore has for its object to provide an improved, more efficient method of preparing and applying bleaches and/or disinfectants, the method further guaranteeing the quality of the produced and applied bleach and/or disinfectant.

The invention provides for this purpose a device for on-site preparation and application of a bleach and/or disinfectant, comprising:
- at least one chemical reactor for forming a bleach and/or disinfectant;
- at least one feed device connected to the reactor for feeding reactants required for the purpose of forming the bleach and/or disinfectant; - at least one discharge device connected to the reactor for discharging the bleach and/or disinfectant formed in the reactor, said at least one discharge device is configured for coupling to a treatment device for objects, in particular textile or food product, for supplying at least a part of the formed bleach and/or disinfectant to the treatment device; and
- at least one flowmeter coupled to the at least one discharge device for measuring the quantity of bleach and/or disinfectant, wherein the at least one chemical reactor comprises at least one detection element for detecting the chemical activity and the device further comprises a processing unit configured to control the at least one flowmeter on the basis of the chemical activity detected by the at least one detection element, and wherein the processing unit is configured to control the at least one flowmeter to measure the quantity of the formed bleach and/or disinfectant in the case a predefined minimum chemical activity is detected by the at least one detection element.

The above device is connectable to a treatment device for objects such as textile, dairy, food and beverage products, and thereby provides the user of the treatment device with the option of preparing bleach and/or disinfectant comprising sanitizing agents and/or cleaning agents at the moment that the use of bleaches and/or disinfectants is desired. This creates a more efficient match between demand and supply of bleach and/or disinfectant. As already described above, the concentration of peracid in peracid-based sanitizing agents decreases in the course of time. Providing a device which is connectable to a treatment device will make it possible to reduce to the minimum the time between preparation of the bleach and/or disinfectant and the eventual application thereof, this enhancing the quality, and the associated treatment process, of the prepared sanitizing agent.

A device for on-site producing and dispensing of cleaning solutions has been disclosed in US 2007/0231220 A1 wherein the in situ production of chlorine dioxide at the point of use has been disclosed. The cleaning solution is produced by reacting chemicals within a dispensing apparatus containing a catalyst or reactant. The formed cleaning solution is then stored in a tank prior to being dispensed.
However, given the on-site preparation of cleaning solutions disclosed in the prior art, the final concentration of the formed bleach and/or disinfectant further depends on the reactants used, the duration of the reaction for preparing the formed bleach and/or disinfectant and the reaction conditions in general, such as the metal ions present in the solvent used (for instance water), the temperature during the reaction and the amounts of reactants used during the forming of bleach and/or disinfectant. In order to guarantee the quality of the formed bleach and/or disinfectant the device of the present invention provides a detection element which detects the chemical activity in the at least one chemical reactor and on the basis of which the flowmeter is controlled. It has thus been observed that a solution comprising a bleach and/or disinfectant also comprises a significant residual fraction which has no sanitizing action. This residual fraction is particularly significant when the formed bleach and/or disinfectant is stored for a longer period. In addition, the quantity of residual fraction will also vary due to variation in the above specified reaction conditions. Detection of only the amounts of reactants added to the reactor is therefore not a representative method of determining the formation of bleach and/or disinfectant, and therefore the quality of the sanitizing agent. It has been found that measuring the amount of formed bleach and/or disinfectant which measurement is at least controllable on the basis of the chemical activity detected by the at least one detection element, provides for an improved norm for determining and guaranteeing the quality of the prepared sanitizing agent. Such a method also provides the option of determining the operating costs for the user of the treatment device on the basis of the actually produced quantity of bleach and/or disinfectant. The operating costs will therefore correlate with the final quality and/or quantity of the actually used sanitizing agent. It is also possible, on the basis of the chemical activity detected by the detection element, to optimize the process for preparing the sanitizing agent on-site while taking into account the ambient conditions prevailing on-site.

In an embodiment variant according to the present invention the at least one chemical reactor comprises a reaction vessel such as a mixing vessel in which two or more reactants are mixed in order to form a bleach and/or disinfectant or an intermediate product thereof. Diverse reaction vessels can be coupled to each other in order to provide the option of forming diverse intermediate products before producing the end product. In another embodiment variant according to the present invention the at least one chemical reactor comprises a reactor in line. Such a reactor is particularly advantageous for the forming of a bleach and/or disinfectant by means of a continuous process. Such a reactor can be configured to supply reactants at different locations. The device according to the present invention is particularly suitable for preparing a sanitizing agent based on peracid, in particular peroxy acids with a carbon chain length varying from 1 to 8. The device according to the present invention is preferably suitable for preparing peracid wherein the reactants preferably comprise an acyl donor, a peroxide and a base. The acyl donor is preferably formed by a compound with the formula (1): 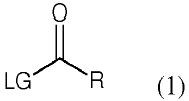 wherein
LG is a leaving group formed by an amide, amine, ester, anhydride or halide; and
R is formed by a hydrogen atom (H) or alkyl. The reaction (2) of this compound with hydrogen peroxide and/or peroxide ions is shown as follows:

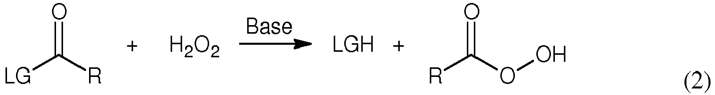

It has been found that acyl donors with a chemical structure corresponding to the above formula are particularly suitable for the manufacture of a peracid. The type of peracid formed depends on the chemical structure of the acyl donor, and particularly on the nature of R (hydrogen or alkyl group).

A peroxide is preferably selected from the group consisting of hydrogen peroxide or peroxide ions. A base is preferably selected from the group consisting of alkali metal hydroxide, such as sodium hydroxide or potassium hydroxide, or other types of base, such as alkanolamines (for instance monoethanolamine), N'N-diisopropylethylamine, 2,6-di-tert-butylpiridine and 1,8-Diazabicycloundec-7-ene.

The device according to the present invention is preferably suitable for preparing peracetic acid. In such a reaction the acyl donor can preferably be selected from the group consisting of acetyl donor compounds such as triacetin, diacetin, ethylene glycol diacetate, acetylsalicylate, ethylene glycol monoacetate and/or pentaerythritol tetra-acetate. The device can further be configured to prepare peracids wherein the acyl donor is preferably selected from the group consisting of oxybenzoic acid esters, such as DOBA, benzene sulfonate esters, such as NOBS and LOBS, sorbitan esters, such as sorbitan sesquioctanoate, sorbitan octanoate, sorbitan hexanoate, sorbitan sesquihexanoate, sorbitan decanoate and sorbitan sesquidecanoate, monosaccharide esters, such as glucose esters, and/or glycerol esters, such as trioctanoin and polysaccharide esters, such as sucrose esters.

The stability of the bleach and/or disinfectant to be manufactured in the device can be further improved by adding sequestrants configured to chelate metal ions which will be present in the solution. Tests have shown that the sequestrants co-act relatively selectively with heavy metal ions. A determined sequestrant can be highly suitable for the purpose of chelating a determined type of metal ion, such as an iron ion, and hardly or not at all suitable for the purpose of chelating another type of metal ion, such as a copper ion. In order to isolate as many heavy metal ions as possible in order to guarantee as far as possible the stability in the obtained aqueous solution, it is usually advantageous for a plurality of sequestrants to be added to the at least one chemical reactor of the device according to the present invention. At least two sequestrants are preferably selected here which are suitable for chelating mutually differing heavy metal ions. As many heavy metal ions as possible, naturally present in traces (<5%, generally <1%) in alkali metal hydroxide and/or the solvent applied (for instance water), can in this way be chelated.

The sequestrants are preferably formed by at least one sequestrant, generally in acidic state or in the form of a salt, selected from the group consisting of: HEDP (Hydroxyethylidene-diphosphonic acid, PBTC (Phosphonobutane-tricarboxylic acid), DTPMP (Diethylenetriamine pentamethylene phosphonic acid), Sodium heptogluconate or Sodium gluconate, MGDA (Methylglycinediacetic acid), EDDS (Ethylenediamine disuccinic acid), ATMP (Amino trimethylene phosphonic acid), IDS (Tetrasodium Iminodisuccinate), GLDA (N, N-bis (carboxymethyl)glutaminic acid, EDTA (Ethylenediaminotetraacetic acid), EDTMP (Ethylenediaminetetramethylenephosphonic acid), HEDTA (Hydroxyl-ethylenediaminetriacetic acid), DPA (Dipicolinic acid), citrates, STPP (sodium tripolyphosphate), silicates, polymerized phosphonates, stannates, magnesium sulfate, CMI (carboxymethyl inulin) and derivatives thereof, sodium polyaspartate, polysuccinimide, and/or polyacryl acids or copolymers associated therewith. The greatest stability in the solution is obtained with the sequestrants HEDP, DTPMP, a polymerized phosphonate, ATMP and IDS. Addition of these preferred sequestrants to the at least one chemical reactor according to the present invention results in hardly any degradation of the formed peracid after 15 minutes following the initial reaction. The 15-minute period is representative for application of the formed peracid, such as peracetic acid, as constituent in a treatment device such as a cleaning installation, for instance for food products, textile, (medical) instruments and so on. When one of the above stated preferred sequestrants is applied, the stability of formed peracid will be substantially 96-98%, this meaning that 96-98% of the actually formed peracid concentration (100%) is maintained.

In order to guarantee the quality of the bleach and/or disinfectant, the at least one chemical reactor comprises at least one detection element for detecting the chemical activity. Chemical activity is understood in this context to mean not only the thermodynamic activity of a reaction. The detection element can for instance be configured to detect (a predefined minimum change in) the temperature, the pressure, the concentration of reactants and/or formed intermediate or end product, the pH value, the polarization, the colour, the conductivity or combinations thereof. It is emphasized that this is a non-limitative list of possible measurable process parameters. The flowmeter can then be controlled, i.e. whether or not the quantity of bleach and /or disinfectant formed is measured, on the basis of the chemical activity detected by the detection element, for instance the change in temperature.

The flowmeter according to the present invention is configured to measure the quantity of formed bleach and/or disinfectant. Such a measurement can for instance be performed by measuring the throughfeed volume or measuring the throughfeed time. The flowmeter can also take the form of a sensor for measuring for instance the concentration of the bleach and/or disinfectant for throughfeed. The flowmeter can be controlled, i.e. regulated, on the basis of the chemical activity detected by the detection element such that the mixture formed in the chemical reactor is carried further through the discharge device connectable to the treatment device or that the mixture formed in the chemical reactor is discharged to for instance a waste tank. In such an embodiment variant the flowmeter comprises a controllable throughflow controller. The flowmeter preferably comprises one or more valves for controllable throughfeed of the mixture formed in the chemical reactor.

As already stated above the device further comprises a processing unit which is configured to control the flowmeter on the basis of the chemical activity detected by the at least one detection element. Such a processing unit preferably comprises an intelligent control unit, such as a programmable logic unit (PLC) and is preferably connected to the at least one detection element and the flowmeter. The processing unit can be set such that the flowmeter is activated in the case of a minimal chemical activity detected by the detection element, i.e. the flowmeter discharges formed disinfectant and/or bleach further through the discharge device. The minimal chemical activity can be defined in different ways. The processing unit is preferably provided with predefined limit values which are representative of the chemical activity to be detected.

The processing unit is configured to control the flowmeter on the basis of whether or not the predefined limit value has been exceeded. The processing unit is thus configured to activate the flowmeter when a minimal chemical activity is detected by the detection element. The quantity of formed bleach and/or can for instance be measured through activation of the flowmeter.

The processing unit can also be configured to receive an activating signal generated by the treatment device. Such an activating signal can be generated in the case the supply of bleach and/or disinfectant to the treatment device is desired. This activating signal can also comprise a specific quantity of bleach and/or disinfectant to be supplied. The processing unit can then control the production of bleach and/or disinfectant on the basis of this signal and produce only the required quantity of sanitizing agent.

In an embodiment of the present invention the processing unit is configured to process information collected by the flowmeter. Such information can for instance relate to the quantity of the formed bleach and/or disinfectant. Such information can however also relate for instance to the quantity of bleach and/or disinfectant discharged to for instance a waste tank. Such a configuration provides the device, and also the use of the device, with the option of analysing the information relating to the quantity of bleach and/or disinfectant to be supplied to and/or supplied to the treatment device. Not only can the quantity of the formed bleach and/or disinfectant be determined on the basis of such an analysis, it also provides the user with the option of determining the quality of the formed bleach and/or disinfectant.

In an embodiment variant according to the present invention the flowmeter comprises at least two controllable throughflow controllers. In such an embodiment variant the first throughflow controller is configured to discharge disinfectant and/or bleach formed in the chemical reactor to the treatment device. The second throughflow controller is preferably configured to discharge disinfectant and/or bleach formed in the chemical reactor to for instance a waste tank and/or other discharge not connectable to the treatment device. Both throughflow controllers are preferably at least controllable on the basis of the chemical activity detected by the at least one detection element. Both throughflow controllers are more preferably connected to the above described processing unit.

In respect of guaranteeing the quality of the sanitizing agent, it is possible to opt to provide the processing unit with predetermined values. The device can be provided with warning means which generate a warning signal at the moment that the detected formed bleach and/or disinfectant falls outside the predetermined values. In addition, it is possible to provide the device with a closure which can close the coupling between the device and the treatment device when a sanitizing agent is prepared which does not meet the minimum quality requirements.

In a further embodiment according to the present invention the processing unit is configured to calculate operating costs on the basis of the information collected by the flowmeter. The information collected by the flowmeter preferably comprises information relating to determining of the number of batches of manufactured bleach and/or disinfectant by measuring the quantity of formed sanitizing agent and/or cleaning agent (for instance in mass or volume), and/or information relating to the concentration of bleach and/or disinfectant, and therefore the quality, of the manufactured batch by determining the percentage of bleach and/or disinfectant that is present. As already stated above, it has been found that calculation of the operating costs on the basis of the supplied reactants is not a representative method in view of the fact that the quality of the sanitizing agent can differ per use and per location. A constant quality of the sanitizing agent and/or cleaning agent to be manufactured can be guaranteed by means of detecting the above described information using a flowmeter controllable on the basis of detected chemical activity, this flowmeter being coupled to the at least one discharge device. The device of the present invention therefore also provides a method wherein the user of the treatment device is invoiced only on the basis of the produced quantities of bleach and/or disinfectant instead of on the basis of the starting materials supplied.

In an additional embodiment the processing unit is configured for exchange of information with an external network. The exchange, i.e. transmitting and/or receiving, of information is particularly advantageous for remote monitoring of the performance of the device and for adjustment where necessary. An external network preferably comprises an external server which is coupled by means of a wireless connection to the processing unit of the device. Such an exchange of information with an external network also provides the option of determining the remaining quantities of reactants on the basis of the quantities of bleach and/or disinfectant produced. In an embodiment of the present invention the device is provided with a control panel where the user of the device has the possibility of entering the quantities of supplied reactants. The remaining quantities are calculated on the basis of these data. It is however also possible to provide the device with measuring means which monitor the quantity of remaining reactant.

As already stated above, the processing unit is configured to control the flowmeter on the basis of a predefined minimum chemical activity. The predefined minimum chemical activity is preferably a minimum change in temperature, in pressure, in pH value, in degree of polarization, in colour change, in conductivity, in concentration of formed intermediate or end product, in concentration of reactants or combinations thereof. In addition to the predefined minimum chemical activity, the chemical activity can also comprise a predetermined maximum chemical activity. Such limit values in respect of the maximum chemical activity can for instance be used in order to generate a warning signal or to interrupt the operation of the device when they are exceeded.

The flowmeter preferably detects the quantity of bleach and/or disinfectant, such as the number of batches and/or fraction of formed bleach and/or disinfectant, by making use of a counter and/or on the basis of supply time and/or supply volume.

The at least one detection element can further be coupled to the at least one feed device for feeding reactants to the reactor for the purpose of detecting the quantity of reactant supplied to the reactor. The at least one detection element can also be configured to send electronic signals for the purpose of the remaining quantity of reactant.

In an embodiment variant of the present invention the at least one detection element and/or the flowmeter is configured for exchange of information with an external network. As already stated above, the exchange, i.e. transmitting and/or receiving, of information is particularly advantageous in remotely monitoring and where necessary adjusting the performance of the device.

In an embodiment variant of the present invention the device further comprises a feed device connected to the reactor for feeding a solvent to the reactor. The solvent preferably comprises water, although it is also possible to opt for another solvent subject to the bleach and/or disinfectant to be produced.

In an embodiment variant the device according to the present invention further comprises a dosing device placed between the discharge device and treatment device. Such a dosing device controls the delivery of bleach and/or disinfectant to a treatment device coupled to the device. It is also possible to provide the device according to the present invention with a storage tank, or throughflow tank, placed between the discharge device and treatment device for the purpose of temporarily storing too rapid a production or overproduction of bleach and/or disinfectant. Both the dosing device and/or the storage tank can be provided with supply means for supplying stabilizers, such as the above described sequestrants, for the purpose of increasing the stability of the sanitizing agent and/or cleaning agent present in the dosing device and/or the storage tank.

The flowmeter is preferably positioned between the at least one chemical reactor and the above stated dosing device and/or storage tank. When use is made of a storage tank, it is also possible to place an optional second flowmeter downstream of the storage tank, i.e. between the storage tank and the treatment device. The storage tank preferably comprises at least one second detection element for detecting the chemical activity in the storage tank. The optional second flowmeter is more preferably controlled on the basis of the chemical activity detected by the at least second detection element. Similarly as already described above, the processing unit can be configured to control the second flowmeter on the basis of the chemical activity detected by the second detection element.

The discharge device of the present invention can also be provided with a first discharge element coupled to the treatment device for the purpose of supplying at least a part of the formed bleach and/or disinfectant to the treatment device, and a second discharge element for draining at least a part of the formed bleach and/or disinfectant. Such a construction of the discharge device provides for the possibility of directly discharging sanitizing agent of too low a quality formed in the reactor without such low-quality sanitizing agent being added to the treatment device. The second discharge element is preferably coupled to a waste tank. If use is made of a storage tank as described above, the discharge device can be provided with such a first and second discharge element upstream and/or downstream of the storage tank.

In an embodiment variant of the device according to the present invention the flowmeter is placed at a position lying between the reactor on the one hand and the first and second discharge element on the other. Such a construction provides the option of measuring the quality of the bleach and/or disinfectant formed in the reactor using the detection element before determining whether to feed the formed bleach and/or disinfectant to the treatment device.

The device according to the present invention is preferably connectable to a treatment device, wherein the treatment device is suitable for cleaning and sanitizing installations used for instance in the food industry and/or the treatment of textile, food product, medical equipment and/or crockery.

The device according to the present invention is therefore suitable for application in the textile industry, such as the laundry industry, as well as in medical treatment centres and professional kitchens.

The invention further provides a method for on-site preparation and application of a bleach and/or disinfectant according to claim 9.

In an embodiment variant the method also comprises step f), comprising of calculating operating costs to be charged on the basis of the quantity measured during step e) of the formed. bleach and/or disinfectant.

In a further embodiment variant of the present invention the method further comprises of holding in storage the formed bleach and/or disinfectant discharged in step c) before guiding at least a part of the formed bleach and/or disinfectant to the treatment device.

The method according to the present invention can also comprise of adding a solvent to the reactor. The method can also be activated after receiving an activating signal generated by the treatment device.

The invention further provides a system comprising at least one device according to the present invention and at least one treatment device for objects, in particular textile or food product, connected to the at least one device, wherein the discharge device of the device is coupled to the treatment device such that formed bleach and/or disinfectant can be supplied via the discharge device to the treatment device.

The system of the present invention can further comprise a dosing device and/or storage tank, i.e. throughflow tank, placed between the device according to the present invention and the treatment device connected to the device.

In an embodiment variant according to the present invention the system comprises a server coupled to the device for receiving, analysing and/or transmitting data generated by the device, in particular the flowmeter and/or the processing unit. As already indicated above, such a system provides the option of remotely monitoring and controlling the device forming part of the system. The device can also be configured to receive signals generated by the server, such as warning signals in the case of a shortage of reactants or signals relating to improvement in the quality of the bleach and/or disinfectant to be produced.

The system of the present invention preferably comprises a treatment device located in a laundry, kitchen, food processing industry or medical treatment centre.

The invention further provides for the use of a flowmeter according to claim 15.

The invention will be elucidated on the basis of non-limitative exemplary embodiments shown in the following figures. Herein:
figure 1 shows a schematic view of a device according to the invention, and
figure 2 shows a schematic view of a system according to the invention comprising the device according to figure 1.

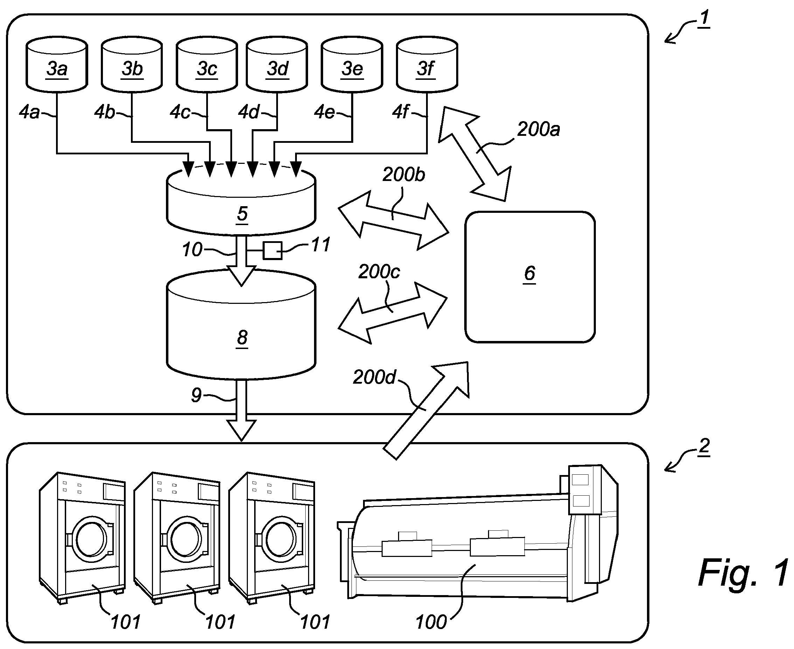

Figure 1 shows a schematic view of a device 1 according to the invention. In this exemplary embodiment device 1 is configured to prepare a bleach and/or disinfectant which can be added to a washing process of a treatment device 2, for instance an industrial tunnel washer 100 or industrial drum washing machine 101 for textile. Device 1 comprises a plurality of supply containers 3a, 3b, 3c, 3d, 3e, 3f. The first supply container 3a is at least partially filled with an acyl donor, preferably triacetin. The second supply container 3b is preferably filled with water, such as for instance demineralized water or tap water with a recommended temperature of a maximum of 30°C. The third supply container 3c is at least partially filled with hydrogen peroxide and the fourth supply container 3d is at least partially filled with a strong base, preferably sodium hydroxide. Supply containers 3e and 3f provide the option of premixing diverse reactants for the purpose of forming an intermediate product. A typical volume of supply containers 3a, 3b, 3c amounts to between 0.1 and 2 kg. Supply containers 3a-3f are coupled (releasably) by means of feed devices 4a-4f via a plurality of valves (not shown) to a chemical reactor 5. By opening the valves the different starting materials can be guided into reactor 5 for the purpose of preparing the sanitizing agent and/or cleaning agent. The valves are preferably controlled here by a processing unit 6, usually formed by a PLC (Programmable Logic Controller). Addition of the starting materials to reactor 5 preferably takes place in dosed manner and also in a predefined sequence, wherein the base is generally added as last starting material to reactor 5. The valves can also function as pre-dosing elements for the purpose of pre-dosing a desired quantity of starting material to be added to reactor 5. Separate pre-dosing elements can optionally be applied. The pre-dosing elements can be provided with a float for metering the desired quantity of starting material. Emptying of the pre-dosing elements can take place under time-control (for instance 10-15 seconds) and/or for instance by means of weighing starting material present in the pre-dosing elements. In this embodiment reactor 5 has a typical size of between 5 and 20 litres. Reactor 5 is provided with a mixing device (not shown) as well as one or more probes (not shown) coupled to processing unit 6 for the purpose of monitoring, among other values, the temperature, pH, redox potential and so on of the exothermic reaction mixture in reactor 5.

Processing unit 6 is preferably configured to receive signals 200a, 200b, 200c, 200d, preferably electrical signals, which signals 200a, 200b, 200c come from the different components 3a-3f, 5, 8 of device 1 or signals 200d which come from treatment device 2. Processing unit 6 can also be configured to transmit signals 200a, 200b, 200c to the different components 3a-3f, 5, 8 of device 1. Such signals 200a, 200b, 200c generated by processing unit 6 can be used to control device 1.

Water is preferably added first to reactor 5. If no water is present in the reaction vessel, the software of the PLC 6 can be programmed to stop the preparation process. The water is added at the end of the preceding cycle/preparation without cleaning the reaction vessel 5. The other starting materials are added only when a new preparation begins.

The water is preferably dosed by using a flowmeter (not shown) or per volume by making use of a dosing unit (not shown) with a level trip or time-controlled valve with an accuracy of more than 99%. This can also be seen as a pre-dosing element (not shown).

The overall accuracy of all dosed products is more than 99% in order to be able to guarantee the quality of the sanitizing agent and/or cleaning agent produced on site. It is possible to monitor the consumption on site of sanitizing agent and/or cleaning agent of each laundry connected to device 1.

Device 1 can be programmed such that, when the water level is correct and verified by a measurement, reactants are added to reactor 5 and the mixture will be stimied. The base is preferably added as final reactant, whereby the pH value of the mixture will be increased, although it must be ensured that a pH value of a maximum of 11.5 is not exceeded here. During mixing of the different reactants for the purpose of forming a peracid-based bleach and/or cleaning agent an exothermic reaction takes place wherein the pH value of the mixture also falls below 11. Because the energy released by the reaction is itself a reaction product, it is sufficient to measure the temperature increase

in each batch size in order to verify the quality of the reaction. If the difference in temperature during the preparation does not correspond to the specifications provided, the system will stop the preparation and discharge the mixture to the waste tank. If the temperature increase is too great and amounts to more than 40°C, the preparation is also stopped and the mixture discharged to the waste tank.

The difference in temperature is defined as the difference between the temperature of the solution following the reaction minus the temperature before the reaction. When the difference in temperature is validated, mixing takes place for two minutes and the peracid generated on-site in reactor 5 goes directly to a throughflow tank 8 which is coupled via conduit 10 to the reactor and where the generated peracid can be stored for a maximum of two hours before use in treatment device 2. Throughflow tank 8 enables a more accurate dosing to treatment device 2 and makes it possible to connect a plurality of washing units to treatment device 2. Throughflow tank 8 is thus coupled by means of a conduit 9 to treatment device 2, whereby peracid prepared on-site can be immediately employed for use in the washing process taking place in the adjacent or nearby treatment device 2.

The final temperature of the product is ideally below 28°C. Below this temperature both the yield of the reaction and the stability of the final product are optimal. This is the reason why in some cases, when the temperature of the incoming water is too high (more than 18°C), for instance during the summer, it is possible to envisage cooling the water to for instance 15°C. This cooling need not necessarily be controlled by the system and can take place at another location on site.

Depending on the moment at which the product is going to be used (immediately following preparation or 1-2 hours after preparation), the ratio of peracetic acid and H₂O₂ varies between 6.0-3.0.

Device 1 also comprises one or more flowmeters 11, one of which is coupled here to conduit 10. Flowmeters 11 can also be positioned elsewhere downstream of reactor 5. Flowmeter 11 is configured to detect the quantity of bleach and/or disinfectant generated, in particular the quantity of bleach and/or disinfectant supplied to treatment device 2. The actual consumption of the bleach and/or disinfectant can in this way be monitored and recorded using the PLC 6. This can take place with relatively high accuracy (more than 99%). One or more flowmeters can also be applied for rejected preparations of bleach and/or disinfectant guided via a discharge (not shown) out of reactor 5 in the direction of a waste tank (not shown). The rejected batches can thus also be recorded.

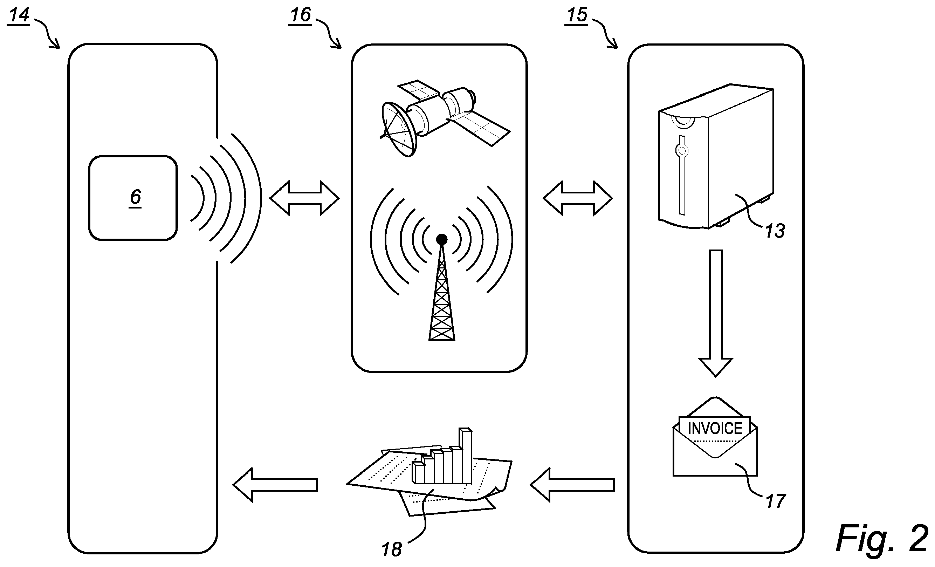

Figure 2 shows a schematic view of a system for invoicing of an administrator 14 of a treatment device 2 by an administrator 15 of device 1. Figure 2 shows a system in which some or all information detected by the PLC 6 can be transmitted via a wireless network 16 to a server 13 of the administrator 15 of device 1, usually formed by the supplier of the bulk materials. Administrator 15 of device 1 will then be able to calculate the actual operating costs and the costs relating to rejected batches by means of server 13, and on the basis of this information the costs 17 can be charged to an administrator 14 of treatment device 2, who will receive an invoice 18 herefor. Administrator 14 will generally only pay for the validated batches. This is therefore an output-based charging of costs, wherein payment will only be made for the realized consumption. The system as shown in figure 2 further comprises the option for the administrator 15 of device 1 of providing the PLC 6 of device 1 with diverse information, such as software updates, method improvements and so on, by means of a wireless connection 60.

The system as shown in figure 2 comprising device 1 therefore provides the option for administrator 15 of device 1 of monitoring the consumption of starting materials by each individual customer, such as administrator 14 of treatment device 2. The administrator 15 of device 1 hereby has the option of providing the end user proactively with starting materials when the remaining quantity of starting materials falls below a predetermined level.

The invention further relates to a method for manufacturing a bleach and/or disinfectant comprising sanitizing agent and/or cleaning agent, preferably using the above described device. The invention provides for this purpose a method for preparing a bleach and/or disinfectant comprising of preparing an aqueous mixture of at least a peroxy acid and hydrogen peroxide and/or peroxide ions, wherein the aqueous mixture is prepared by allowing reaction in water of (I) at least one acyl donor with (ii) hydrogen peroxide and/or peroxide ions, wherein added to the water are: (iii) at least one base and optionally (iv) at least one sequestrant.

The reaction which occurs following mixing of the acyl donor and the hydrogen peroxide and/or the peroxide ions is a nucleophilic acyl substitution reaction which can be performed particularly quickly and efficiently in an alkaline environment, preferably with an initial pH lying between 10 and 11.5. This alkaline environment is created by adding the at least one strong base, preferably an alkali metal hydroxide such as sodium hydroxide (NaOH) or potassium hydroxide (KOH), which functions as strong base. Instead of alkali metal hydroxide other types of base can also be used, such as alkanolamines, N'N-diisopropylethylamine, 2,6-di-tert-butylpiridine and 1,8-Diazabicycloundec-7-ene.

The aqueous mixture can be formed by mixing the starting materials water, acyl donor and hydrogen peroxide and/or peroxide ions, wherein these starting materials can be added in any random sequence. The base is however preferably added last.

Our own research has shown however that the impurities (contaminants) present in commercially obtainable alkali metal hydroxides usually comprise heavy metal ions such as iron ions, nickel ions, manganese ions and copper ions, and/or transition metal ions such as zinc ions or cadmium ions, which have an adverse effect on the stability of the formed peracid (peroxy acid). One or more sequestrants are therefore added to the aqueous solution or dispersion in order to chelate the metal ions of heavy metals and/or transition metals by chelation, whereby the relatively high, non-equilibrium concentration of peracid obtained can be maintained relatively well. Such metal ions can however also have another origin and for instance already be present (in traces) in the applied water, whereby application of the one or more sequestrants for such metal ions is advantageous. Heavy metal ions are understood below to mean ions originating from heavy metals and/or transition metals.

The diverse suitable acyl donors and sequestrants have already been described at length above. The method according to the present invention particularly envisages the manufacture of a peracid, in particular peroxy acids with a carbon chain length varying from 1 to 8. The method according to the present invention more particularly provides for the manufacture of peracetic acid (PAA).

In a preferred embodiment the at least one acyl donor is selected from a group of acyl donors consisting of: acetyl precursors or alkyl precursors. Both precursors configured to form peracids are preferably acetyl esters, for instance diacetin, triacetin, diacetin ethylene glycol diacetate, ethylene glycol monoacetate, pentaerythritol tetra-acetate and acetylsalicylate. Most recommended will generally be triacetin. Triacetin is relatively inexpensive, readily available and configured to generate with one molecule three molecules of PAA, which as already noted acts particularly effectively as bleach and disinfectant.

Other suitable acyl precursors are preferably selected from the group consisting of: oxybenzoic acid esters, such as DOBA, benzene sulfonate esters, such as NOBS and LOBS, sorbitan esters, such as sorbitan sesquioctanoate, sorbitan octanoate, sorbitan hexanoate, sorbitan sesquihexanoate, sorbitan decanoate and sorbitan sesquidecanoate, monosaccharide esters, such as glucose esters, and/or glycerol esters, such as trioctanoin and polysaccharide esters, such as sucrose esters.

The acidity of the obtained aqueous mixture is an important factor in the stability of the formed peracid, in particular PAA. A high pH is on the one hand particularly advantageous in bringing about a rapid perhydrolysis of the acyl donor, particularly triacetin. An (initial) pH higher than 10.5 is preferably applied here, and more preferably a pH higher than 11, and more preferably lower than 11.5. This relates to the acidity at the moment the chemical reaction begins wherein the peracid is formed. During and after the chemical reaction the pH will generally decrease to some extent (a pH lying below 11) as a result of the forming of peracetic acid. Applying the alkali hydroxide helps to keep the pH at the desired level. However, a relatively high pH generally still has an adverse effect on the stability of the formed PAA, in particular the peracetate ion from PAA, and even accelerates the decomposition of the formed PAA (alkaline hydrolysis), wherein the peracetate with water is converted to acetic acid or at least acetate and hydrogen peroxide. At lower pH values close to the pK_{z} of PAA, the PAA will decompose spontaneously. The optimum acidity range lies between the two limits, wherein PAA (or other peracid) will react as a result of alkaline hydrolysis or spontaneous decomposition. It has been found that, if the aqueous mixture does not exceed a pH of 11.5 during the forming of peracid, in particular PAA, a particularly advantageous concentration of peracid, in particular PAA, can be obtained.

As already stated above, under the influence of the forming of peracid, such as PAA, the pH will fall after a period of time to a pH value lower than 11. This relatively low pH value has the advantage that determined active constituents, such as enzymes and/or hydrophobic (lipophilic) peracids, such as phtalimidoperoxy hexanoic acid (PAP), can be applied in particularly effective manner. PAP generally functions optimally, just as enzymes, at a pH of between 7.0 and 9.5. When the composition obtained according to the invention is applied, wherein lipophilic bleach and/or enzymes are/have been added, it is advantageous for the pH to be decreased until it falls within the above stated pH range of 7.0 to 9.5, preferably 8.5 to 9.5, in order to allow the additives to function as well as possible.

The concentration of hydrogen peroxide and/or peroxide ions preferably lies between 1% w/w and 10% w/w. The concentration of hydrogen peroxide and/or peroxide ions preferably lies between 1% w/w and 5% w/w. Different types of hydrogen peroxide can be used, these types varying from 19% w/w to 49.4% w/w hydrogen peroxide. Dosages can subsequently be modified in order to obtain a desired concentration percent by weight.

The concentration of acyl donor preferably lies between 1% w/w and 10% w/w. The concentration of acyl donor preferably amounts to 1% w/w to 5% w/w.

The molar ratio of the hydrogen peroxide (or peroxide ions derived therefrom) and the (reactive groups of the) acyl donor preferably lies between 1 : 2 and 4 : 1 (peroxide : acyl donor). The molar ratio of the peroxide and the acyl donor is preferably > 0.5 : 1. The molar ratio of the peroxide and the acyl donor is more preferably > 1 : 1. Most recommended is a molar ratio of the peroxide and the acyl donor substantially equal to 1.3 : 1. A molar ratio of > 1.3 : 1 will not generally result in further increase in the reaction efficiency. The acyl donor can have more than one reactive group which can react with the peroxide (in accordance with a nucleophilic acyl substitution reaction). When the acyl donor has multiple reactive groups, the above stated molar ratios must then be modified accordingly. Triacetin for instance thus has three reactive groups which can react with peroxide ions, whereby the ideal molar ratio will come to lie at or around 3.9 : 1 (peroxide : triacetin).

The concentration of base is preferably 0.3% w/w to 5% w/w. In a particularly advantageous embodiment of the method according to the present invention the base is added as last component to the aqueous mixture. It has been found that such an addition sequence results in an exceptionally advantageous yield of the formed peracid, in particular PAA. It has also been found that, by applying a sequence wherein the base is added last to the aqueous mixture, the formed peracid, such as PAA, can be held and stored in stable state. The above stated method particularly provides a method for manufacturing a bleach and/or disinfectant, which formed bleaches and/or disinfectant can be stored for 1 to 2 hours before being used without the concentration of the formed bleach and/or disinfectant decreasing during storage.

### Example

The dosing containers 3a-3f are connected to packagings comprising chemicals, including water, triacetin, hydrogen peroxide and sodium hydroxide, the dosing containers 3a-3f being connected via a feed pump to these chemical packagings. The administrator of the treatment device has the option of adapting the quantity of the product to be prepared in accordance with the consumption and the capacity of the cleaning installation. This quantity can be a fixed quantity, although it is also possible to adjust this quantity, for instance when one or more cleaning installations must be used simultaneously. In contrast to the other ingredients, the water can be added directly to the reaction vessel. The quantities of chemicals to be supplied can be metered with a calibrated electronic flowmeter or a level trip indicator. Device 1 is preferably connected to a throughflow tank 8. When the level of the product in the throughflow tank is lower than a defined threshold value, a new batch is prepared.

A peracetic acid-based sanitizing agent and/or cleaning agent is prepared by feeding water to reaction vessel 5. For optimum mixing of the water and the other components still to be added, the water and the mixture for forming are stirred by means of a mixer. Triacetin is then added to one of the dosing containers 3a-3f. The weight and/or the volume of triacetin is determined in the dosing container. Hydrogen peroxide is added to the same or a separate dosing container. Sodium hydroxide is then added to a separate dosing container. The different quantities of components are recorded by the PLC 6. If all pre-dosages are correct, triacetin and hydrogen peroxide are added to reactor 5. After stirring (for instance for 30 seconds), sodium hydroxide is added to the aqueous reaction mixture, wherein the pH value of the aqueous mixture amounts to a maximum of 11. During the perhydrolysis of triacetin and the forming of peracetic acid (PAA) a temperature increase is observed simultaneously with a pH reduction. The mixture is stirred for two minutes and, when the temperature difference is validated with a predefined maximum temperature difference, the batch is deemed as being in accordance with the specifications and transferred to throughflow tank 8. If the temperature difference does not lie within the preferred range of 8-10°C, an alarm notification is generated and the product is rejected and discharged. Device 1 is configured to receive an electronic signal from treatment device 2 when PAA has to be supplied to treatment device 2. After receiving such a signal, device 1 will supply the demanded quantity of PAA from throughflow tank 8 to the relevant treatment device 2. Device 1 is further configured to receive an electronic signal generated from throughflow tank 8 when the quantity of PAA lies below a determined value. After receiving such a signal, device 1 will begin production of PAA in accordance with the above described method. The quantity of PAA added to the washing process is measured by means of flowmeter 11 and charged at a later point in time to the administrator 14 of treatment device 2.

It will be apparent that the invention is not limited to the exemplary embodiments shown and described here but that numerous variants which will be self-evident to the skilled person in this field are possible within the scope of the appended claims.

## Claims

1. Device (1) for on-site preparation and application of a bleach and/or disinfectant, comprising:
- at least one chemical reactor (5) for forming a bleach and/or disinfectant;
- at least one feed device (4) connected to the reactor (5) for feeding reactants required for the purpose of forming the bleach and/or disinfectant;
- at least one discharge device (10) connected to the reactor (5) for discharging the bleach and/or disinfectant formed in the reactor (5), said at least one discharge device (10) is configured for coupling to a treatment device (2) for objects, in particular textile or food product, for supplying at least a part of the formed bleach and/or disinfectant to the treatment device (2); and
- at least one flowmeter (11) coupled to the at least one discharge device (10) for measuring the quantity of the formed bleach and/or disinfectant,
**characterized in that** the at least one chemical reactor (5) comprises at least one detection element for detecting the chemical activity and the device (1) further comprises a processing unit (6) configured to control the at least one flowmeter (11) on the basis of the chemical activity detected by the at least one detection element, wherein the processing unit (6) is configured to control the at least one flowmeter (11) to measure the quantity of the formed bleach and/or disinfectant in the case a predefined minimum chemical activity is detected by the at least one detection element.

2. Device (1) according to claim 1, wherein the processing unit (6) is configured to process information collected by the at least one flowmeter (11).

3. Device (1) according to claim 1 or 2, wherein the processing unit (6) is configured for exchange of information with an external network.

4. Device (1) according to any of the preceding claims, wherein the device (1) further comprises a storage tank (8) placed between the discharge device (10) and the treatment device (2).

5. Device (1) according to any of the preceding claims, wherein the at least one flowmeter (11) comprises a controllable throughflow controller configured to dose a controllable quantity of formed bleach and/or disinfectant to the treatment device (2).

6. Device (1) according to any of claims 1-4, wherein the device (1) further comprises a dosing device placed between the discharge device (10) and the treatment device (2).

7. Device (1) according to any of the preceding claims, wherein the discharge device (10) is provided with a first discharge element coupled to the treatment device (2) for the purpose of supplying at least a part of the formed bleach and/or disinfectant to the treatment device (2), and a second discharge element for draining at least a part of the formed bleach and/or disinfectant.

8. Device (1) according to claim 7, wherein the at least one flowmeter (11) is placed at a position lying between the reactor (5) on the one hand and the first and second discharge element on the other.

9. Method for on-site preparation and application of a bleach and/or disinfectant, in particular with the device (1) according to any of the preceding claims, comprising the processing steps of:
a) adding reactants required for forming a bleach and/or disinfectant to a reactor (5);
b) allowing the reactants to react so as to form the bleach and/or disinfectant; and
c) discharging the formed bleach and/or disinfectant from the reactor, wherein at least a part of the formed bleach and/or disinfectant is guided to a treatment device (2) for objects, in particular textile or food product,
wherein the method further comprising the processing steps of:
- during process step b), detecting the chemical activity; and
- during process step c), measuring the quantity of the formed bleach and/or disinfectant in the case a minimum chemical activity is detected in processing step b).

10. Method according to claim 9, wherein the method further comprises of holding in storage the formed bleach and/or disinfectant discharged in step c) before guiding at least a part of the formed bleach and/or disinfectant to the treatment device (2).

11. System comprising:
- at least one device (1) according to any of claims 1-8; and
- at least one treatment device (2) for objects, in particular textile or food product, connected to the at least one device (1),
wherein the discharge device (10) of the device is coupled to the treatment device (2) such that formed bleach and/or disinfectant can be supplied via the discharge device (10) to the treatment device (2).

12. System according to claim 11, wherein the system further comprises a dosing device and/or storage tank (8) placed between the device (1) and the treatment device (2) connected to the device (1).

13. System according to claim 11 or 12, wherein the system comprises a server (13) coupled to the device (1) for receiving, analysing and/or transmitting data generated by the device (1), in particular the at least one flowmeter (11) and/or the processing unit (6).

14. System according to claim 13, wherein the device (1) is configured to receive signals generated by the server (13).

15. Use of a flowmeter (11), in particular in the device (1) according to any of claims 1-8, for measuring the quantity of formed bleach and/or disinfectant, wherein the flowmeter (11) is configured to measure the quantity of the formed bleach and/or disinfectant in the case a minimum chemical activity is detected.

## Patentansprüche

1. Vorrichtung (1) für die Vor-Ort-Herstellung und Anwendung eines Bleich- und/oder Desinfektionsmittels, umfassend:
- wenigstens ein chemisches Reaktionsgefäß (5) zum Herstellen eines Bleich- und/oder Desinfektionsmittels;
- wenigstens eine Zuführvorrichtung (4), die mit dem Reaktionsgefäß (5) verbunden ist, zum Zuführen von Reaktionsmitteln, die für den Zweck der Herstellung des Bleich- und/oder Desinfektionsmittels erforderlich sind;
- wenigstens eine Entladevorrichtung (10), die mit dem Reaktionsgefäß (5) verbunden ist, zum Entladen des in dem Reaktionsgefäß (5) hergestellten Bleich- und/oder Desinfektionsmittels, wobei die wenigstens eine Entladevorrichtung (10) zum Koppeln mit einer Behandlungsvorrichtung (2) für Gegenstände, insbesondere Textil- oder Lebensmittelprodukte, gestaltet ist, um der Behandlungsvorrichtung (2) wenigstens einen Teil des hergestellten Bleich- und/oder Desinfektionsmittels zuzuführen; und
- wenigstens einen Durchflussmesser (11), der mit der wenigstens einen Entladevorrichtung (10) gekoppelt ist, um die Menge des hergestellten Bleich- und/oder Desinfektionsmittels zu messen,
**dadurch gekennzeichnet, dass** das wenigstens eine chemische Reaktionsgefäß (5) wenigstens ein Nachweiselement zum Nachweisen der chemischen Aktivität umfasst und die Vorrichtung (1) ferner eine Prozessoreinheit (6) umfasst, die dafür gestaltet ist, den wenigstens einen Durchflussmesser (11) auf der Grundlage der von dem wenigstens einen Nachweiselement nachgewiesenen chemischen Aktivität zu steuern, wobei die Prozessoreinheit (6) dafür gestaltet ist, den wenigstens einen Durchflussmesser (11) zu steuern, die Menge des hergestellten Bleich- und/oder Desinfektionsmittels in dem Fall zu messen, dass durch das wenigstens eine Nachweiselement eine vordefinierte chemische Mindestaktivität nachgewiesen wird.

2. Vorrichtung (1) gemäß Anspruch 1, wobei die Prozessoreinheit (6) dafür gestaltet ist, von dem wenigstens einen Durchflussmesser (11) aufgenommene Informationen zu verarbeiten.

3. Vorrichtung (1) gemäß Anspruch 1 oder 2, wobei die Prozessoreinheit (6) zum Austauschen von Informationen mit einem externen Netzwerk gestaltet ist.

4. Vorrichtung (1) gemäß einem der vorstehenden Ansprüche, wobei die Vorrichtung (1) ferner einen Vorratstank (8) umfasst, der zwischen der Entladevorrichtung (10) und der Behandlungsvorrichtung (2) angeordnet ist.

5. Vorrichtung (1) gemäß einem der vorstehenden Ansprüche, wobei der wenigstens eine Durchflussmesser (11 ) eine steuerbare Durchflusssteuerung umfasst, die dafür gestaltet ist, eine steuerbare Menge an hergestelltem Bleich- und/oder Desinfektionsmittel zu der Behandlungsvorrichtung (2) zu dosieren.

6. Vorrichtung (1) gemäß einem der Ansprüche 1-4, wobei die Vorrichtung (1) ferner eine Dosiervorrichtung umfasst, die zwischen der Entladevorrichtung (10) und der Behandlungsvorrichtung (2) angeordnet ist.

7. Vorrichtung (1) gemäß einem der vorstehenden Ansprüche, wobei die Entladevorrichtung (10) mit einem ersten Entladeelement versehen ist, das mit der Behandlungsvorrichtung (2) gekoppelt ist, zu dem Zweck, der Behandlungsvorrichtung (2) wenigstens einen Teil des hergestellten Bleich- und/oder Desinfektionsmittels zuzuführen, und mit einem zweiten Entladeelement zum Ablassen wenigstens eines Teils des hergestellten Bleich- und/oder Desinfektionsmittels.

8. Vorrichtung (1) gemäß Anspruch 7, wobei der wenigstens eine Durchflussmesser (11) an einer Position angeordnet ist, die zwischen dem Reaktionsgefäß (5) einerseits und dem zweiten Entladeelement andererseits liegt.

9. Verfahren für die für die Vor-Ort-Herstellung und Anwendung eines Bleich- und/oder Desinfektionsmittels, insbesondere mit der Vorrichtung (1) gemäß einem der vorstehenden Ansprüche, umfassend die Verfahrensschritte:
a) Zugeben von Reaktionsmitteln, die für die Herstellung eines Bleich- und/oder Desinfektionsmittels erforderlich sind, zu einem Reaktionsgefäß (5);
b) Umsetzenlassen der Reaktionsmittel, um das Bleich- und/oder Desinfektionsmittel zu bilden; und
c) Entladen des hergestellten Bleich- und/oder Desinfektionsmittels aus dem Reaktionsgefäß, wobei wenigstens ein Teil des hergestellten Bleich- und/oder Desinfektionsmittels zu einer Behandlungsvorrichtung (2) für Gegenstände, insbesondere Textil- oder Lebensmittelprodukte, geleitet wird,
wobei das Verfahren ferner die Verfahrensschritte umfasst:
- während des Verfahrensschritts b) Nachweisen der chemischen Aktivität; und
- während des Verfahrensschritts c) Messen der Menge an hergestelltem Bleich- und/oder Desinfektionsmittel in dem Fall, dass bei dem Verfahrensschritt b) eine chemische Mindestaktivität nachgewiesen wird.

10. Verfahren gemäß Anspruch 9, wobei das Verfahren ferner das Bevorraten des bei Schritt c) entladenen hergestellten Bleich- und/oder Desinfektionsmittels umfasst, bevor wenigstens ein Teil des hergestellten Bleich- und/oder Desinfektionsmittels zu der Behandlungsvorrichtung (2) geleitet wird.

11. System, umfassend:
- wenigstens eine Vorrichtung (1) gemäß einem der Ansprüche 1-8; und
- wenigstens eine Behandlungsvorrichtung (2) für Gegenstände, insbesondere Textil- oder Lebensmittelprodukte, die mit der wenigstens einen Vorrichtung (1) verbunden ist,
wobei die Entladevorrichtung (10) der Vorrichtung mit der Behandlungsvorrichtung (2) gekoppelt ist, so dass hergestelltes Bleich- und/oder Desinfektionsmittel über die Entladevorrichtung (10) der Behandlungsvorrichtung (2) zugeführt werden kann.

12. System gemäß Anspruch 11, wobei das System ferner eine Dosiervorrichtung und/oder einen Vorratstank (8) umfasst, die/der zwischen der Vorrichtung (1) und der mit der Vorrichtung (1) verbundenen Behandlungsvorrichtung (2) angeordnet ist.

13. System gemäß Anspruch 11 oder 12, wobei das System einen an die Vorrichtung (1) gekoppelten Server (13) zum Empfangen, Analysieren und/oder Übertragen von Daten umfasst, die von der Vorrichtung (1), insbesondere dem wenigstens einen Durchflussmesser (11) und/oder der Prozessoreinheit (6), erzeugt werden.

14. System gemäß Anspruch 13, wobei die Vorrichtung (1) dafür gestaltet ist, von dem Server (13) erzeugte Signale zu empfangen.

15. Verwendung eines Durchflussmessers (11), insbesondere in der Vorrichtung (1) gemäß einem der Ansprüche 1-8, zum Messen der Menge von hergestelltem Bleich- und/oder Desinfektionsmittel, wobei der Durchflussmesser (11) dafür gestaltet ist, die Menge des gebildeten Bleich- und/oder Desinfektionsmittels in dem Fall zu messen, dass eine chemische Mindestaktivität nachgewiesen wird.

## Revendications

1. Dispositif (1) destiné à la préparation et à l'application in situ d'un agent blanchissant et/ou d'un désinfectant, comprenant :
au moins un réacteur chimique (5) destiné à former un agent blanchissant et/ou un désinfectant,
au moins un dispositif d'application (4) relié au réacteur (5) dans le but de fournir des réactifs requis pour former l'agent blanchissant et/ou le désinfectant,
au moins un dispositif d'évacuation (10) relié au réacteur (5) pour évacuer l'agent blanchissant et/ou le désinfectant formé dans le réacteur (5), ledit ou lesdits dispositifs d'évacuation (10) étant configurés pour être couplés à un dispositif de traitement (2) pour des objets, en particulier textile ou produit alimentaire, afin de fournir au moins une partie de l'agent blanchissant et/ou du désinfectant formé au dispositif de traitement (2), et
au moins un débitmètre (11) couplé à ce ou à ces dispositifs d'évacuation (10) afin de mesurer la quantité de l'agent blanchissant et/ou du désinfectant formé,
**caractérisé en ce que** le ou les réacteurs chimiques (5) comprennent au moins un élément de détection destiné à détecter l'activité chimique, et le dispositif (1) comprenant en outre une unité de traitement (6) configurée pour commander le ou les débitmètres (11) sur la base de l'activité chimique détectée par le ou les éléments de détection,
dans lequel l'unité de traitement (6) est configurée pour commander le ou les débitmètres (11) pour qu'ils mesurent la quantité d'agent blanchissant et/ou de désinfectant formé dans le cas où une activité chimique minimale prédéfinie est détectée par le ou les éléments de détection.

2. Dispositif (1) selon la revendication 1, dans lequel l'unité de traitement (6) est configurée pour traiter les informations recueillies par le ou les débitmètres (11).

3. Dispositif (1) selon la revendication 1 ou la revendication 2, dans lequel l'unité de traitement (6) est configurée pour échanger des informations avec un réseau externe.

4. Dispositif (1) selon l'une quelconque des revendications précédentes, dans lequel le dispositif (1) comprenant en outre un réservoir de stockage (8) placé entre le dispositif d'évacuation (10) et le dispositif de traitement (2).

5. Dispositif (1) selon l'une quelconque des revendications précédentes, dans lequel le ou les débitmètres (11) comprennent un contrôleur d'écoulement contrôlable configuré pour doser une quantité contrôlable d'agent blanchissant et/ou de désinfectant formé vers le dispositif de traitement (2).

6. Dispositif (1) selon l'une quelconque des revendications 1 à 4, dans lequel le dispositif (1) comprenant en outre un dispositif de dosage placé entre le dispositif d'évacuation (10) et le dispositif de traitement (2).

7. Dispositif (1) selon l'une quelconque des revendications précédentes, dans lequel le dispositif d'évacuation (10) est muni d'un premier élément d'évacuation couplé au dispositif de traitement (2) dans le but de fournir au moins une partie de l'agent blanchissant et/ou du désinfectant formé au dispositif de traitement (2), ainsi qu'un second élément d'évacuation destiné à drainer au moins une partie de l'agent blanchissant et/ou du désinfectant formé.

8. Dispositif (1) selon la revendication 7, dans lequel le ou les débitmètres (11) sont placés à une position située entre le réacteur (5) d'un côté et les premier et second éléments d'évacuation de l'autre.

9. Procédé destiné à la préparation et à l'application in situ d'un agent blanchissant et/ou d'un désinfectant, en particulier avec le dispositif (1) conforme à l'une quelconque des revendications précédentes, comprenant les étapes de traitement suivantes :
a) l'ajout à un réacteur (5) de réactifs requis pour former un agent blanchissant et/ou un désinfectant,
b) la permission faite aux réactifs de réagir de façon à ce qu'ils forment l'agent blanchissant et/ou le désinfectant, et
c) l'évacuation de l'agent blanchissant et/ou du désinfectant formé hors du réacteur, au moins une partie de l'agent blanchissant et/ou du désinfectant formé étant guidée vers un dispositif de traitement (2) pour des objets, en particulier textile ou produit alimentaire,
dans lequel le procédé comprenant en outre les étapes de traitement suivantes :
- pendant l'étape de traitement b), la détection de l'activité chimique, et
- pendant l'étape de traitement c), la mesure de la quantité d'agent blanchissant et/ou de désinfectant formé dans le cas où une activité chimique minimale est détectée lors de l'étape de traitement b).

10. Procédé selon la revendication 9, dans lequel le procédé comprend en outre le maintien en stockage de l'agent blanchissant et/ou du désinfectant formé évacué lors de l'étape c) avant de guider au moins une partie de l'agent blanchissant et/ou du désinfectant formé vers le dispositif de traitement (2).

11. Système comprenant :
- au moins un dispositif (1) conforme à l'une quelconque des revendications 1 à 8, et
- au moins un dispositif de traitement (2) pour des objets, en particulier textile ou produit alimentaire, relié à ce ou à ces dispositifs (1),
dans lequel le dispositif d'évacuation (10) du dispositif est couplé au dispositif de traitement (2) de telle sorte que l'agent blanchissant et/ou le désinfectant formé puisse être fourni au dispositif de traitement (2) par l'intermédiaire du dispositif d'évacuation (10).

12. Système selon la revendication 11, dans lequel le système comprend en outre un dispositif de dosage et/ou un réservoir de stockage (8) placé entre le dispositif (1) et le dispositif de traitement (2) relié au dispositif (1).

13. Système selon la revendication 11 ou la revendication 12, dans lequel le système comprend un serveur (13) couplé au dispositif (1) afin de recevoir, d'analyser et/ou de transmettre des donnés générées par le dispositif (1), en particulier par le ou les débitmètres (11) et/ou l'unité de traitement (6).

14. Système selon la revendication 13, dans lequel le dispositif (1) est configuré pour recevoir des signaux générés par le serveur (13).

15. L'utilisation d'un débitmètre (11), en particulier dans le dispositif (1) conforme à l'une quelconque des revendications 1 à 8, afin de mesurer la quantité d'agent blanchissant et/ou de désinfectant formé, dans lequel le débitmètre (11) étant configuré pour mesurer la quantité d'agent blanchissant et/ou de désinfectant formé dans le cas où une activité chimique minimale est détectée.
